# EUROPEAN PATENT APPLICATION

(11) **EP 2 327 711 A1**
(43) Date of publication of application: **01.06.2011**
(21) Application number: 09806479.3
(22) Date of filing: 31.07.2009
(51) Int. Cl.: C07K 1/14

(54) **METHODS FOR REMOVING PROLAMINS FROM WHEAT THAT ARE TOXIC TO THOSE SUFFERING FROM COELIAC DISEASE**

(30) Priority: 07.08.2008 ES 200802393
(71) Applicant: Universitat Rovira I Virgili, 43003 Tarragona (ES); ICREA, Institució Catalana de Recerca I Estudis Avançats, 08010 Barcelona (ES)
(72) Inventor: O'SULLIVAN, Ciara, Kathleen, E-43007 Barcelona (ES); BERMUDO REDONDO, Mª, Carmen, E-43007 Tarragona (ES); KATAKIS, Ioannis, E-43007 Tarragona (ES)
(74) Representative: Carpintero Lopez, Francisco
(86) International application number: PCT/ES2009/070328
(87) International publication number: WO 2010/018290

(57) **Abstract**

The present invention relates to a composition for removing gliadins and glutanins, which are prolamins that are toxic to patients suffering from coeliac disease, contained in heat-processed and non-heat-processed foods. The composition of the invention comprises a reducing agent for irreversible reduction of disulphide bridges and a solvent with a high boiling point and/or high dielectric constant in a buffer with a pH between 4 and 9. The invention also relates to the method for removing gluten (gliadins and glutenins) from food products, which is compatible with an enzyme-linked immunosorbant assay (ELISA), that allows detection of hydrolized and non-hydrolized gluten (gliadins and glutenins). Lastly, the invention relates to a kit for implementing said method.

## Description

### FIELD OF THE INVENTION

This invention refers in its general context to the analysis of food, heat processed and non-heat processed, for its gluten (gliadins and glutenins) content, and more specifically to a procedure to extract gluten (gliadins and glutenins) from food products, compatible with an enzyme linked immunosorbent assay (ELISA), capable of detecting hydrolysed and non-hydrolysed gluten (gliadins and glutenins).

### BACKGROUND OF THE INVENTION

Coeliac disease is an inflammatory disease of the upper small intestine and results from gluten ingestion in genetically susceptible individuals, and is the only life long nutrient-induced enteropathy.

Coeliac disease is triggered in susceptible individuals by the ingestion of gluten prolamins from wheat, barley, rye and possibly oats causing histological changes in the small intestine mucosa, leading to a mal-absorption syndrome [1]. It has been known since 1953 that the condition exacerbated when patients eat foods containing wheat flour and subsequently rye and barley, a man-made wheat rye cross, called triticale and possibly oats, were also implicated. The only treatment for CD is a strict gluten-free diet, with the longer the individual fails to adhere to this diet, the greater the chance of developing malnutrition and other complications and all of the disturbances revert when a strict gluten-free diet is established [2].

Gluten is the protein content in wheat, rye, oats, barley and maize and may be divided into glutenin and gliadin (alcohol soluble fraction). Glutenins are insoluble in water or alcohol but may become soluble once interchain disulphide bonds are reduced. They may be further divided into high molecular weight (HMW) and low molecular weight (LMW) fractions and gliadin may be subdivided into α, γ and w fractions, according to their N-terminal amino acid sequences. The corresponding proteins in rye, barley and oats are known as secalins, hordeins and avenins, respectively.

Coeliac patients present a broad range of sensitivity to gluten intake, with clinical manifestations to minimal amounts of gliadin reported [3]. Consequently, to certify gluten-free products, the use of highly sensitive assays is mandatory. The European Union, World Health Organisation and Codex Alimentarius require reliable measurement of the wheat prolamin, gliadin (as well as the other cereal prolamins), rather than all wheat-derived proteins, which include albumins, globulins and starch granule proteins [4].

A major problem in the production of 'gluten-free' raw materials and in the production of gluten free food is the contamination throughout the process and quite often ingredients and products that began life as gluten free become contaminated and dangerous for consumption by a coeliac disease sufferer.

The official limits described in the Codex Draft Revised Standard (2000) are 20 p.p.m. for foodstuffs naturally gluten-free, 200 p.p.m for foodstuffs rendered gluten-free; solid foodstuffs on a dry matter basis, and liquid foodstuffs on the basis of the original product. Progress in the development of a reliable method of analysis for gluten is difficult.

Currently available kits for gluten measurement:
- are time-consuming, indirect sandwich assays [5-8],
- cannot detect gliadin equivalently in the various cereals [5, 7],
- are not specific for coeliac disease toxic gliadin [8, 9],
- do not detect hydrolysed forms of gliadin [6, 10] and
- are destroyed by those reducing agents commonly used to extract gluten from food (which denature antibodies and enzyme labels) [7, 10].

A reliable method to measure the gluten content of food items has been therefore notably lacking over the last few years.

At present, gluten is measured by epitope-dependent methods, such as ELISA, which use monoclonal or polyclonal antibodies. A useful ELISA technique to quantify the gluten contents of food products is described in Bermudo et al (2005) (64).

Traditionally it was the gliadin fraction of wheat gluten that was thought to be toxic. Little information existed concerning the glutenins, due to difficulties in their separation and purification. Recent evidence suggests that certain glutenin peptides and recombinant glutenin sub-units [17] can stimulate isolated coeliac small intestinal T cells in vitro. The implication is that these are toxic in vivo. However in vivo data is at present restricted to one article [18]. This study showed a toxic effect in vivo in 4 out of 4 coeliac patients challenged with a mix of four high molecular weight glutenin subunits. Little data is at present available concerning the toxicity of the low molecular weight glutenins, however it must be considered possible that these proteins are also toxic.

Gliadins are readily extracted from raw foodstuffs using ethanol but this only results in a co-extraction of a small percentage of glutenins, and a stronger extraction is required involving the use of reducing agents to solubilise the glutenins. This is because glutenins are polymers that are not readily extracted until interchain disulphide bonds are broken. However, these conditions are also required for the extraction of gliadins and glutenins from cooked foods. When gliadins are heated sulphur molecules present in the alpha and gamma, but not omega gliadins, form disulphide bonds, so that the majority of the gliadins become polymers with the same solubility problems as glutenins. So that, routine methods for extraction of gluten from heat-processed food products are based on the use of alcohols, such as ethanol and propanol mixed with reducing agents such as mercaptoethanol, dithiothreitol, dissociating agents such as urea, sodium dodecyl sulphate, guanidine hydrochloride, and mixtures of these. The resulting compositions used for extraction are toxic, flammable, unstable and have a pungent odour.

Therefore, in the light of the recent discovery that HMW glutenins also exacerbate coeliac disease, it is essential to have a universal protocol that can be used to extract gliadins and glutenins from both raw and heat processed food.

Several extraction protocols have been reported for the extraction of gliadins and glutenins from foodstuffs. Ethanol, 50-70% v/v [19-27] and propanol, 50-70% v/v [24-33] have been commonly used for the extraction of gliadins. Lithium chloride has also been used for extraction of gliadins [34] as well as dimethylsulfoxide (DMSO) [27, 35, 36] and dimethylformamide [27, 37]. Following the Osborne protocol, which involves sequential extraction of the different proteins present in gluten, glutenins have been extracted with reducing agents, such as mercaptoethanol (1-2% w/v) [27, 31, 38] and dithiothreitol (0.1-2% w/v) [24, 25, 28, 30, 32, 33, 39-43], acetic acid (0.7% w/v) [31, 33, 44] and dissociating agents such as urea (2-8 M) [27, 32, 37, 41], guanidine hydrochloride (2-6M) [45] and SDS (1-2%) [45, 46]. The extraction procedures used were basically the same involving rapid vortex and a period of extraction (ranging from 15 to 120 minutes) followed by centrifugation (for a period of 15 to 60 minutes), normally at room temperature, although there are some reports of elevating the temperature to 60°C [28, 31, 41, 43]. Either Tris (hydroxymethyl)aminomethane (Tris) buffer (0.05 M-0.08 M, pH 7.5-8.0), borate buffer (0.05 M, pH 8.5) or sodium phosphate buffer (0.05 M), pH 7.8 were used in the extractions.

Various reports have detailed the use of a mixture of reducing agents and dissociating agents. In one example, Tris buffer plus dissociating agent (8M urea, 6M guanidine hydrochloride or 2% w/v SDS) adjusted to pH 7.5 with nitric acid was used for the extraction of glutenins [19]. The glutenins were then reduced with 5% w/v 2-mercaptoethanol or 0.1% w/v dithiothreitol for 2 hours at room temperature. In another example, 50% v/v propanol was used to extract gliadins, followed by a mixture of 50% v/v propanol, 2M urea and 1% w/v dithiothreitol in Tris buffer (pH 7.5) - extracting in a stepwise format via cycles of 2 minute vortex, 10-20 minutes magnetic stirring and 15 minutes centrifugation [32]. Glutenins were also extracted using a mixture of mercaptoethanol (2% w/v) and urea (2-8 M) in borate buffer (pH 8.5), with extraction being carried out for 60 minutes at room temperature followed by 20 minutes centrifugation [38]. An alternative method using a mixture of reducing and dissociating agents was the use of 9.2 M urea with 1.5% w/v dithiolthreitol, where kernels were solubilised in thus mixture overnight and then centrifuged for 2 hours [47].

Additionally, Wieser et al (1998) described an assay to evaluate the capacity of the extraction of gluten proteins in non-processed and processed food samples. The authors demonstrate that for exhaustive and complete extraction of gliadins from the processed samples, it is necessary to use a reducing agent, and increase the temperature. Using these solutions, they also demonstrate the co-extraction of glutenins and gliadins [50]. In a further article by the same authors, they describe a composition for extraction of glutenins, which uses 50% v/v propanol, urea and DTT in Tris-HCl buffer (pH 7.5) [51]. Nicolas et al describe a protocol where they sequentially extract the various proteins found in gluten, and for the extraction of glutenins, they use a mixture of a dissociating agent (SDS), a reducing agent (mercaptoethanol) in tetraborate buffer, pH 8.5 [53]. Huebner et al describe a sequential extraction of gliadins (using 2 extractions with 70 % v/v ethanol) followed by glutenins (extracted using a a mixture of DTT and urea, or DTT and a combination of urea and guanidine hydrochloride, in PBS buffer, pH 7.7 [54]. Mamone et al describe detection of gliadins and glutenins extracted from different varieties of wheat using liquid chromatography and electrospray mass spectrometry. They extract gliadins with 70% ethanol, and glutenins are extracted in 3 steps using a solution of 50% v/v propanol, urea, DTT and Tris-HCl, pH 7.5. They also include a reduction process to which the glutenin fraction is subsequently exposed for 2 hours at 37°C, consisting of guanidine hydrochloride, EDTA, DTT in Tris-HCl buffer, pH 7.5 [55].

A recent publication involves extraction with 2% w/v mercaptoethanol and 2 M guanidine hydrochloride for 40 minutes at 50°C followed by a rapid vortex and 1 hour incubation at room temperature and finally centrifugation [48]. This publication is the subject of the patent ES 2182698 [49]. The invention describes a composition that includes a reducing agent, (e.g. mercaptoethanol, DTT), a dissociating agent (e.g. Guanidine hydrochloride, SDS), in a buffer (PBS, Tris), of pH between 7 and 8. This composition is useful for the extraction of gluten from processed and non-processed foods. The food sample is incubated with this composition for a period of time varying between 30 and 60 minutes at a temperature between 37 and 50 °C. The solution is then cooled to room temperature and incubated with ethanol (50-70% v/v).

However, document ES 2182698 still does not solve the problem in its totality for the following reasons:
- Inconvenience of use: the reducing agents, mercaptoethanol or DTT, claimed in ES 2182698 are toxic, noxious and pungent agents, considered inconvenient for use by many day to day users.
- The high concentrations in the invention claimed under ES 2182698 affect the enzymatic activity of ELISA marker enzymes and therefore make use impossible for some types of ELISA that are in some cases necessary, particularly the use of competition assays for the detection of hydrolyzates.
- Reliability: it seems that the method declared in ES 2182698 might be a standard to which other methods can be compared; however it does not exhaustively extract all gluten - both gliadins and glutenins
- The detection of hydrolyseds of gluten has, in general, proved unsuccessful mainly due to the fact that the small peptide hydrolyseds are recognised by capture but not by subsequent detection antibodies due to steric hindrance, thus requiring the use of a competitive assay, where the enzyme label comes into direct contact with the extraction buffer, thus necessitating the use of a compatible extraction buffer.

Consequently reliable methods for the measurement and co-extraction of coeliac disease toxic prolamins (gliadins and glutenins) in both hydrolysed and non-hydrolysed forms, in both heat processed and non-heat processed food products, do not exist.

Some recent documents detail other extraction protocols. A recent patent, WO2007/104825, refers to a method for extraction of gluten from heat-processed foodstuffs, which uses a composition consisting of a mixture of a reducing agent (mercaptoethanol, DTT and mixtures of these), and a denaturing agent (SDS, non-ionic detergents such as Tween, and mixtures of these), in a buffer of pH 7-8. The protocol consists of mixing the sample and incubating at a temperature of 30-70 °C, for 10-60 minutes, cooling to room temperature and the denatured and reduced fragments are dissolved in ethanol (50-70 % v/v), centrifuged and the extracted gluten remains in the supernatant [56]. Hurkman et al, extracted the various proteins from flour using a mixture of urea (2M), glycerol (10% v/v), DTT (65mM) in Tris-HCl buffer, pH 8.0 [57]. Dupont et al compare different methods for sequential extraction of gliadins and glutenins, and one example of a buffer he used 50% v/v propanol is used to extract the gliadins, and the glutenins are extracted with 50% v/v propanol containing 25 mM DTT, in both cases the buffer used is Tris-HCl, pH 8 [58].

Further, for particular food samples, it is necessary to perform a defatting step prior to gluten (gliadins and glutenins) extraction. Previous reports indicate the use of petroleum ether [22] or butanol [21] as defatting agents, where samples are mixed with the defatting agent for 15 minutes followed by centrifugation for 10 minutes.

Reducing agents used in known extractions methods, such as mercaptoethanol and dithiothreitol, are sulfhydryl containing reducing agents that are prone to oxidation and rapidly lose their potency and are thus not suitable for long term storage. Moreover, they suffer from disadvantages such as low reduction capacity at low and high pH as well as being toxic, malodorous reagents [60].

Tris(2-carboxyethyl)phosphine, TCEP, is a recently identified reductant that has been reported to be a highly stable and efficient reduction of protein thiols [59]. A recent publication has outlined the possibility of the use of the water soluble TCEP as an attractive alternative to DTT and 2-ME [61]. TCEP has been demonstrated to be a superior reductant to DTT in neutral, acidic and alkaline media [62]. TCEP has also been shown to be a much safer protecting agent for thiol compounds in the presence of metal ions than DTT and additionally acts as a reductant and capping agent, thus eliminating the need for capping agents such as vinylpyridine.

Alcohols routinely used for extraction of gliadins, such as ethanol, have low boiling points, thus limiting the temperature that can be applied during extraction.

In the light of the inconveniences derived from the current gluten extraction methods, the authors of the present invention have carried out a major research study to find a reliable method for the simultaneous extraction of gliadins and glutenins from raw and processed foods.

As a result of such studies, the authors have surprisingly found that the use of a composition consisting of a solvent with high boiling point (higher than 100 °C) and/or high dielectric constant, as compared to other solvents, mixed with an agent capable of the irreversible reduction of disulphide groups, in buffer pH 4-9, with extraction being carried out at greater than 50 °C for a duration of more than 2 minutes, facilitates dissolution of gluten (gliadins and glutenins), allowing the gluten (gliadins and glutenins) content of heat processed and non-heat processed food and beverage products to be quantitatively determined. The use of a dissociating agent is not required.

Based on these progresses, the invention reported here is a far simplified method for the simultaneous extraction of gliadins and glutenins from raw and processed foods. The invention is based on the use of solvents of high boiling points and/or high dielectric constants, which facilitates the application of elevated temperatures during the extraction process, eliminating the need for the use of a dissociating agent.

The features of the composition of the invention allow carrying out an ELISA compatible extraction process of gliadins and glutenins, that allows the rapid and reliable simultaneous extraction of gluten (gliadins and glutenins) from non-heat processed and heat processed food samples and detection of said gluten (gliadins and glutenins) in both its non-hydrolysed and hydrolysed forms.

The composition of the invention for simultaneous extraction of gluten (gliadin and glutenin) extracted from both non heat-processed and heat-processed food products, presents multiples advantages compared with those compositions used in the state of the art. So, this composition has no discernable odour, and is environmentally friendly, in contrast to the pungent odours of reducing and dissociating agents used in routine methods.

Additionally, the composition for extraction of gluten (gliadin and glutenin) from both non-heat processed and heat processed food products, has an increased storage stability compared to compositions routinely used based on ethanol or propanol, with any of the reducing agents mercaptoethanol, dithiothreitol, and/or dissociating agents urea, guanidine hydrochloride, sodium dodecyl sulphate and mixtures of dissociating and reducing agents with different alcohols.

### BRIEF DESCRIPTION OF THE FIGURES

**Figure 1****.** Overview of variables studied for elucidation of optimum extraction protocol. C: Composition to study: 1.25 ml of x% v/v solvent in x mM buffer x, pH x with x% p/v of reducing agent x.
**Figure 2****.** Extraction of prolamins in the presence of different solvents (60% v/v): Sequential gliadin (a) and glutenin (b) extraction, and co-extraction of gliadins and glutenins (c), the latter two in the presence of 1% w/v TCEP. Error bars represent the RSD of 6 extractions carried out in parallel.
**Figure 3****.** Gel electrophoresis of gliadins and glutenins extracted using 60% v/v of different solvents a) ethanol and glycerol; b) propanol, DMSO, DMF, in the presence of 1% w/v TCEP. Four extractions were carried out as is shown in the gel electropherograms.
**Figure 4****.** Co-extraction vs sequential extraction of gluten proteins.
**Figure 5****.** Effect of temperature on gluten proteins extractability.
**Figure 6****.** Co-extraction of gluten proteins in the presence of reducing agents (TCEP, DTT) in 60%v/v glycerol. (M: sample)
**Figure 7****.** Use of dissociating agents. (a) Assay comparing Guanidine Hydrochloride or Urea (1, 3 and 5 M) with 60% glycerol with 0.1% w/v TCEP in HEPES pH 7.4 with 60% glycerol with 0.1% w/v TCEP in HEPES ph 7.4 (b) Visualisation of result in electrophoresis gel.
**Figure 8** (a) Electrophoresis of different defatting agents used to test their effect on extraction of the gluten proteins, (b) Extraction and Bradford detection of protein in flour no defatting (No D); defatting adding simultaneously the extraction solution after the extracting defatted step without drying the sample (D+DS ext); no defatting after 24h (No D (24)); and defatting adding extraction solution after 24h, that is, drying the sample for 24h (D+DS ext (24h)).
**Figure 9****.** Stability studies of extraction solution including DTT (black bar), TCEP (grey) or Mercaptoethanol (white). 300 days of store at room temperature (RT), 4°C and 37°C.
**Figure 10****.** Compatibility of the composition of the invention with a) sandwich and b) competitive ELISA assays using an anitibody against the putative immunodominant epitope. Normalised results are presented with B/Bo representing the absorbance value obtained at specific concentrations normalized by the absorbance obtained at zero concentration.
**Figure 11****.** Gel electrophoresis analysis of gluten extracted at room temperature using 0.1% w/v TCEP in 60% v/v glycerol with 50mM HEPES buffer, pH 7 for (a) non-heat processed samples (raw gluten-containing wheat flour) and (b) heat-processed samples ("pizza base").
**Figure 12****.** Gluten extracted at each extraction from non-heat processed ("flour") and heat-processed samples ("pizza base"), with extraction being carried out at room temperature (i) or at 85 °C (ii), with the quantity extracted seen visually using electrophoresis (a) and represented via absolute quantities (b).
**Figure 13** Comparison of extraction of gluten proteins using (A) 60% ethanol, 250mM mercaptoethanol, 2M Guanidine hydrochloride, with extraction carried out at room temperature; (B) 60% ethanol, 250mM mercaptoethanol, 2M Guanidine hydrochloride, with extraction carried out at 60 ºC; (C) Invented Protocol (IP), Extraction 1; (D) Invented Protocol (IP), Extraction 2. a) Electrophoresis gel and b) Bradford analysis.

### OBJECT OF THE INVENTION

A main objective of the invention refers to a composition that consists of an agent for the irreversible reduction of disulphide agents and a solvent with high boiling point and/or high dielectric constants in a buffer with a pH between 4 and 9.

Another objective of the invention is the process for simultaneous extraction of gliadins and glutenins, from both heat processed and non-heat processed food samples, in a manner that is compatible with ELISA, that comprises extracting the gliadins and glutenins content in the presence of the composition defined in the present invention, with the invention being highly reproducible, allowing quantitative determination of the gluten extracted.

Finally, a third objective of the invention refers to a kit to quantify the gliadins and glutenins contents of both heat processed and non-heat processed food samples, that comprises said composition.

### DETAILED DESCRIPTION OF THE INVENTION

The present invention provides a composition ("composition of the invention"), which consists of an agent for the irreversible reduction of disulphide bonds and a solvent of high boiling point and/or high dielectric constants in a buffer with a pH between 4 and 9, such as HEPES, Tris-HCl, PBS, etc.

The composition of the invention is useful to co-extract coeliac disease toxic prolamins (both gliadins and glutenins) from non-heat processed and heat processed food products.

The agent capable of irreversible reduction of disulphide groups is an agent that does not contain sulfhydryl groups and thus is not prone to oxidation, such as tris (2-carboxyethyl) phosphine (TCEP). The concentration of this non-sulfhydryl containing disulphide group reducing agent is widely variable, only requiring being greater than 0.1 mM.

In a particular embodiment, the solvent used in the composition is selected from DMF, DMSO or glycerol, with a concentration between 30-70% v/v.

In a particular embodiment, the agent capable of irreversibly reducing the disulphide groups is TCEP, the solvent with high boiling point is DMSO, glycerol or DMF, and the buffer is HEPES or PBS, with a pH between 4 and 9.

The composition of the invention is compatible with competitive enzyme linked immunosorbent assays (ELISA), capable of detection of both non-hydrolysed and hydrolysed forms of gluten (both gliadins and glutenins) and does not have any adverse effect on the ELISA (competitive or sandwich) quantification of the total and coeliac disease toxic gluten content of food samples.

Another main aspect of the invention refers to a process for simultaneous extraction of both gliadins and glutenins from a food sample, of both non heat processed and heat processed samples, from herein referred to as the process of the invention, that comprises extracting the gluten contents (gliadin and glutenins) from the sample in the presence of the composition of the invention.

In a particular embodiment, the process comprises a) mixing the food sample with the composition of the invention, and b) incubating the resulting mixture at a temperature of greater than 50 °C, for a period of time of greater than 2 minutes.

This process allows saving considerable time in comparison with the processes previously described, and it presents no differences in extractability, and thus a combined extraction process was suitable.

The food sample to be analysed is prepared by routine methods, when relevant. Flour samples are subjected to a representative sampling procedure to ensure homogeneity. Processed food samples are ground using a pestle and mortar prior to milling and the homogenous ground sample is then accurately weighed.

Subsequently the composition of the invention is introduced into an eppendorf tube, the cap of the tube closed and the tube vortexed for a period of time greater than 10 seconds. The tube is then left to stand for a period of time greater than 10 seconds, before being heated at a temperature of greater than 50 °C, for a period of greater than 2 minutes, before being centrifuged at a speed of a range of at least 3000 rpm, for a period of greater than 5 seconds. The supernatant, containing the extracted gliadins and glutenins, is removed.

Optionally, once the supernatant is removed, fresh composition of the invention is added and the procedure described above repeated. This procedure can be repeated from two to ten times, until complete extraction of gluten (gliadins and glutenins) is achieved.

In a particular embodiment, the process of the invention comprises the use of a solvent with high boiling point selected from DMSO, having a boiling point of 189 °C, glycerol, having a boiling point of 290 °C, and DMF, having a boiling point of 153 °C, allowing elevated temperatures (greater than 50 °C) during the extraction step, thus facilitating enhanced gluten (gliadins and glutenins) extraction.

In a particular embodiment, the extraction of gluten (gliadins and glutenins) is carried out with an aqueous solution of DMSO, glycerol or DMF with TCEP, with an applied temperature during extraction of greater than 50 °C, for a period of time greater than 2 minutes.

The solvent concentration in the aqueous solvent solution can vary within a wide range, and in a particular embodiment the aqueous solution (of DMSO, glycerol or DMF) can vary between 50 and 70% v/v.

The TCEP concentration in the aqueous solution can vary within a wide range, and in one specific application the TCEP concentration should be greater than 0.1 mM.

The extraction process is also compatible with a prior defatting process, previous to the extraction step. In a particular embodiment, the food sample is defatted by mixing with acetone, it is separated by the acetone (centrifugation or filtration), subsequently allowing the acetone to evaporate and immediately using this defatted sample for the extraction process.

The method of the present invention can be used to extract the gliadins and glutenins contained in a heat-processed or non-heat processed food sample followed by the quantification of these prolamins by ELISA. Therefore, in a particular embodiment of the processed of the invention, after the extraction, a quantification step of the extracted gliadins and glutenins using ELISA is carried out (e.g. sandwich or competitive ELISA technique).

In the procedure of the invention, the use of glycerol, DMSO or DMF and TCEP allows application of an elevated temperature, which in turn facilitates complete breakage of disulphide bridges found in glutenins and heat-processed gliadins. In the first extraction, disulphide bridges are broken and about 85% of the produced fragments dissolved in the composition of the invention. In the second and remaining extractions the remaining fragments are dissolved in the composition of the invention. Consistently it is observed that 85% of total gluten is extracted in the first extraction and the remaining 15% in subsequent extractions and a correlation factor base on this observation has been established so that only one extraction is required for quantification of total and coeliac disease toxic gliadins.

This method is suitable for food analyses, particularly, for foodstuffs intended for celiac sufferers and can be applied to microsystem based extraction and subsequent biosensoric detection.

Finally, another main aspect of the present invention refers to a kit comprising the composition of the invention and further all those necessary reagents to perform an ELISA to quantify the gliadins and glutenins contents of both heat processed and non-heat processed food samples. The kit uses either competition or sandwich ELISA assays with antibodies developed directly against HMW glutenins and the putative immunodominant epitope of gliadin.

### EXAMPLES

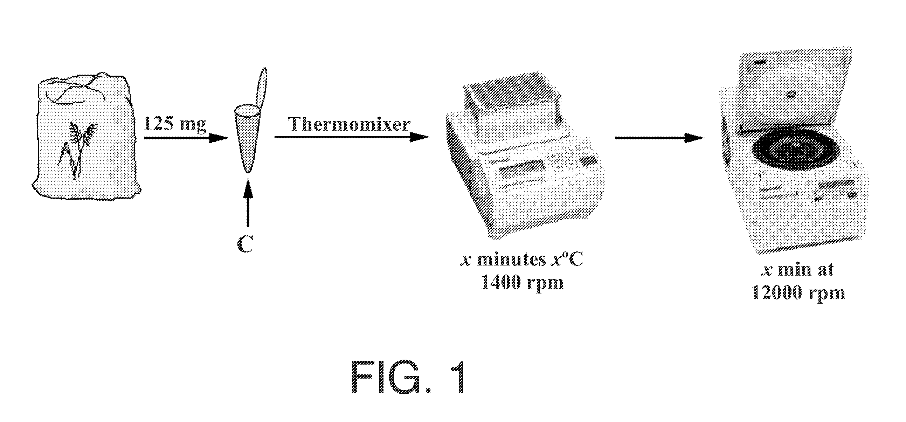

Many experiments were previously carried out resulting in the process of the invention. Specific examples of the application of the process to non-heat treated, heat treated and hydrolysed samples is provided in Examples 1-4. In an initial developmental work, total protein extracted was quantified using a Bradford assay, and visualised using gel electrophoresis. An outline of the extraction scheme tested is shown in Figure 1.

*Bradford Assay:* Using the Pierce Coomasie Plus Bradford Kit 150 µL of extract was added to 150 µL of Coomassie Plus Reagent in a microplate well and mixed well before incubating briefly and measuring the absorbance at 560 nm. Protein concentrations were estimated by reference to the absorbance obtained for a series of standard protein dilutions, varying between 50 µg/ml and 0 µg/ml (gliadin standard for gliadins and glutenins, BSA standard for albumins and globulins), which were assayed alongside the unknown samples.

*Gel Electrophoresis:* For electrophoresis, the samples were precipitated with half that volume of 30% w/v TCA for 1 hr at 4ºC. After centrifugation at 14000 rpm for 10 minutes, the sample pellets were washed 3 times with 200µl acetone and dried under vacuum (<2hr). Upon reconstitution in 10-20 µl SDS running buffer, the samples were heated to 95°C for 5-8 minutes. The samples were loaded in denaturing acrylamide gels (12% resolving, 4% stacking prepared in Tris-SDS buffer pH 8.8 and pH 6.8, respectively) prepared in Tris-SDS buffer pH 6.5. Gels were run in the same Tris-SDS buffer pH 8.3 in BioRad Sub-cell GT Agarose Electrophoresis equipment at room temperature (applying a constant of 100-120 V for gel 10 V/cm). Staining was carried out by immersion of the gels in BioSafe Coommassie (BioRad). Images of the gels were taken using a GelDocTM EQ image analyzer (BioRad).

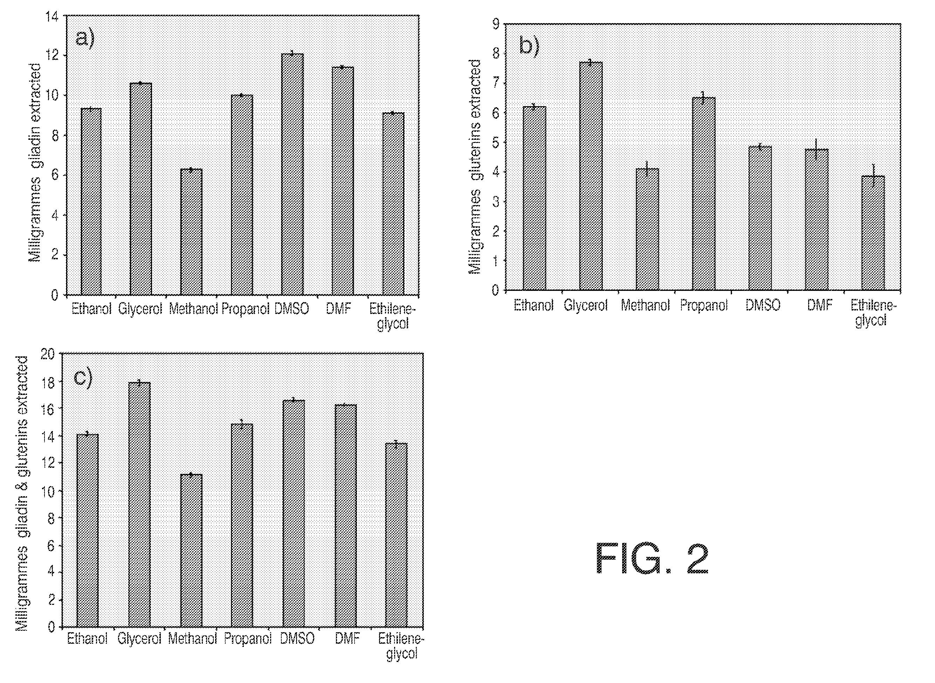

In a first experiment, authors of the invention compared different solvents (60% v/v) for extractability of gluten proteins at room temperature (figure 2). The figure shows the values obtained for each solvent for sequential gliadin (a) and glutenin (b) extraction, and finally the co-extraction of gliadins and glutenins (c), the latter two in the presence of 1% w/v TCEP (error bars represent the relative standard deviation (RSD) of 6 extractions carried out in parallel).

The experiment results showed that DMSO and DMF were the optimum solvents for extraction of gliadins; whilst glycerol was the optimum solvent for extraction of glutenins, when each of the gliadins and glutenins were extracted sequentially. When the prolamins were simultaneously extracted, any of DMSO, DMF or glycerol were optimal, especially when taking into consideration their boiling point, which allows significantly enhanced extraction, especially of HMW glutenins.

**Table 1. Effect of different solvents in gluten proteins extraction (sequential and simultaneous)**

| | **Gliadins (mg)** | **Glutenins (mg)** | **Sum** | **Co-extraction** |
|---|---|---|---|---|
| **Ethanol** | 9.3 | 6.19 | 15.49 | 14.17 |
| **Glycerol** | 10.04 | 7.7 | 17.74 | 17.44 |
| **Methanol** | 6.3 | 4.1 | 10,4 | 11.15 |
| **Propanol** | 10.6 | 6.5 | 17.1 | 14.85 |
| **DMSO** | 12.1 | 4.86 | 16.96 | 16.63 |
| **DMF** | 11.4 | 4.75 | 16.15 | 16.3 |
| **Ethylene Glycol** | 9.12 | 3.87 | 12.99 | 13.4 |

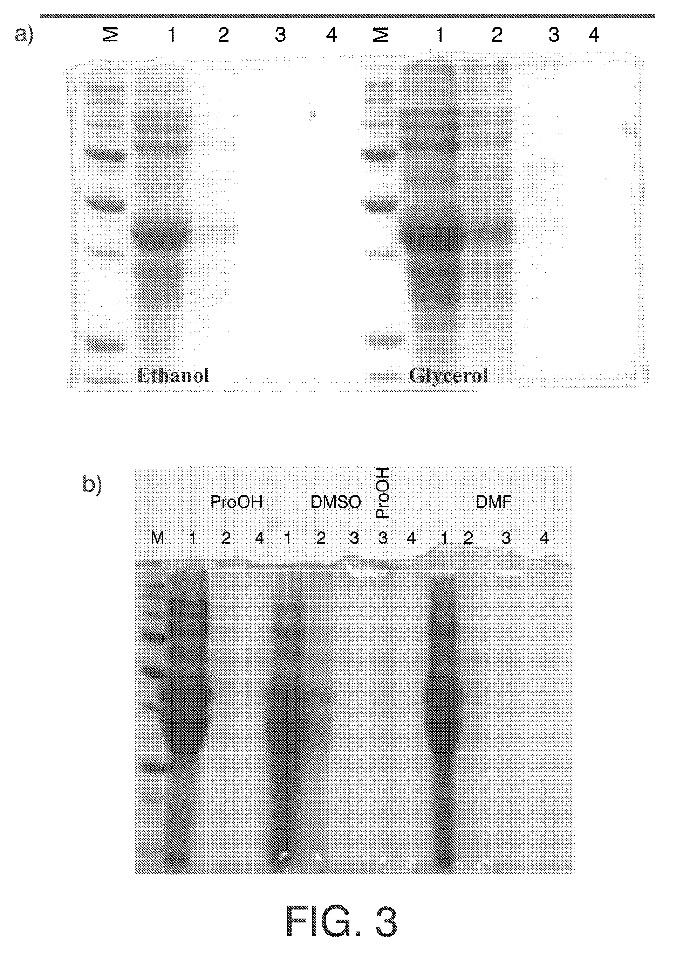

Gliadins and glutenins extracted using 60% v/v of various solvents in the presence of 1% w/v TCEP, at room temperature were visualised in gel electrophoresis (figure 3), where the solvent extractability can be visualized to be glycerol >DMF > DMSO > ethanol > propanol.

In a second experiment, co-extraction vs. sequential extraction of gluten proteins were compared using water and NaCl 0.4 M in HNa₂PO4 0.067 M, for globulins and albumins, respectively. Gliadins were extracted with glycerol 60% w/v and glutenins using glycerol 60% v/v in HEPES 50 mM pH 7.4 with TCEP 0.1% w/v in HEPES 50 mM pH 7.4. Each protein was extracted weighting 125 mg of sample, adding 1.25 ml of extraction solution, mixing at in the thermomixer at 40 °C for 20 minutes at 1400 rpm, and centrifuging for 3 minutes at 12000 rpm.

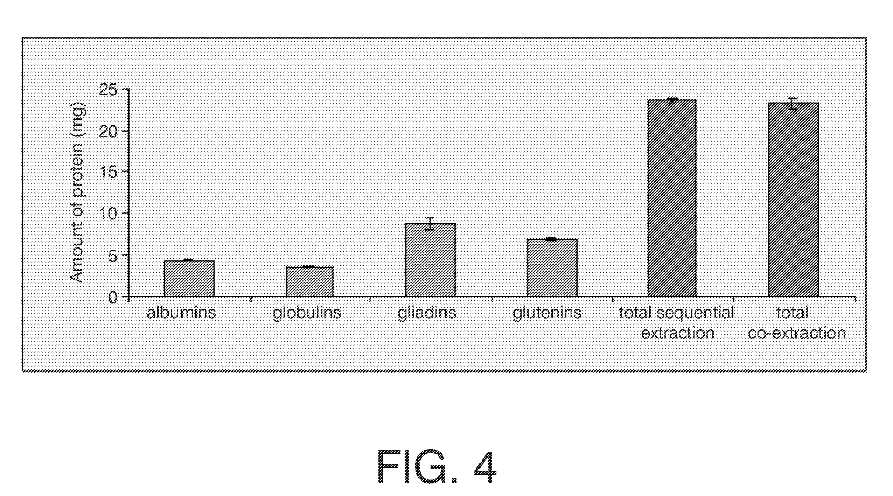

As can be seen in figure 4, no difference in extractability between simultaneous and sequential extraction was observed.

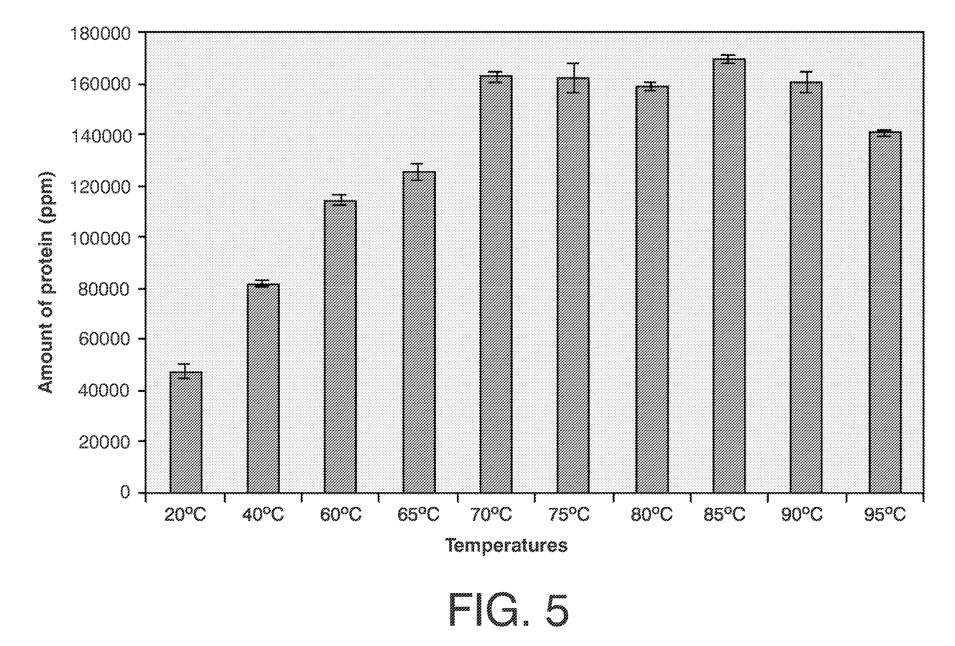

In another experiment, the effect of temperature on prolamins extractability was assayed (figure 5). It showed the need for elevated temperature for extraction of gluten from heat processed samples, with a temperature of 85 °C, being optimal using glycerol as solvent and TCEP as reducing agent.

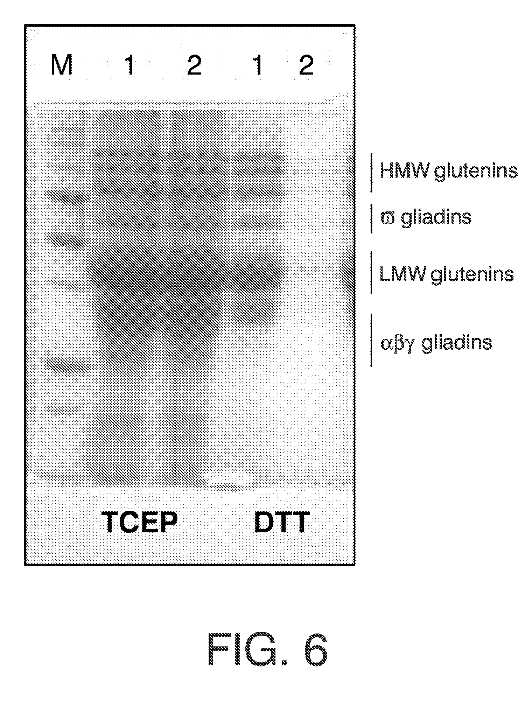

In another experiment, the co-extraction of gluten proteins in the presence of TCEP or DTT, in 60% v/v glycerol, was analyzed. The protein is extracted, weighing 125 mg of sample, adding 1.25ml of the composition of the invention. After extraction for 7 minutes at 85°C, the pellet was centrifuged for 3 minutes at 12000 rpm and the supernatant was used for the electrophoresis analysis (figure 6). This assay demonstrated the differences between the use of different reducing agents, and as can be seen, TCEP is a more effective reducing agent. This is due to the fact that the boiling point of DTT is 125 °C, 2-ME is 157°C, considerably lower than that of TCEP, which is 330°C (thus, the TCEP is able to retain more activity at the applied extraction temperature of 85°C).

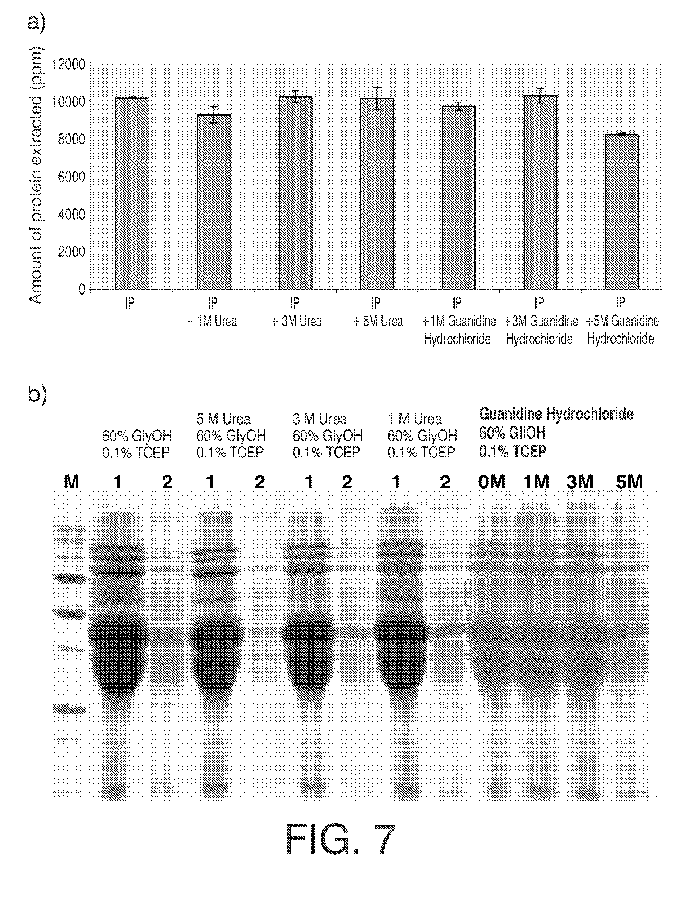

In another experiment, the use of dissociating agents was assayed (figure 7). An assay comparing guanidine hydrochloride or urea (1, 3, 5 M) with 60% glycerol with 0.1% w/v TCEP in HEPES pH 7.4 with 60% glycerol with 0.1% w/v TCEP in HEPES pH 7.4 was developed. The extractions were carried out weighting 125 mg of sample, adding 1.25 ml of extraction solution, mixing at in the thermomixer at 40°C for 20 minutes at 1400 rpm, and centrifuging for 3 minutes at 12000 rpm (figure 7 (a)). Results were visualised in an electrophoresis gel (figure 7 (b)).

This assay demonstrated that in the process of the invention, the addition of dissociating agents does not result in further extraction of gluten from raw or cooked foodstuffs, as the use of elevated temperature is enough to exhaustively extract the gluten.

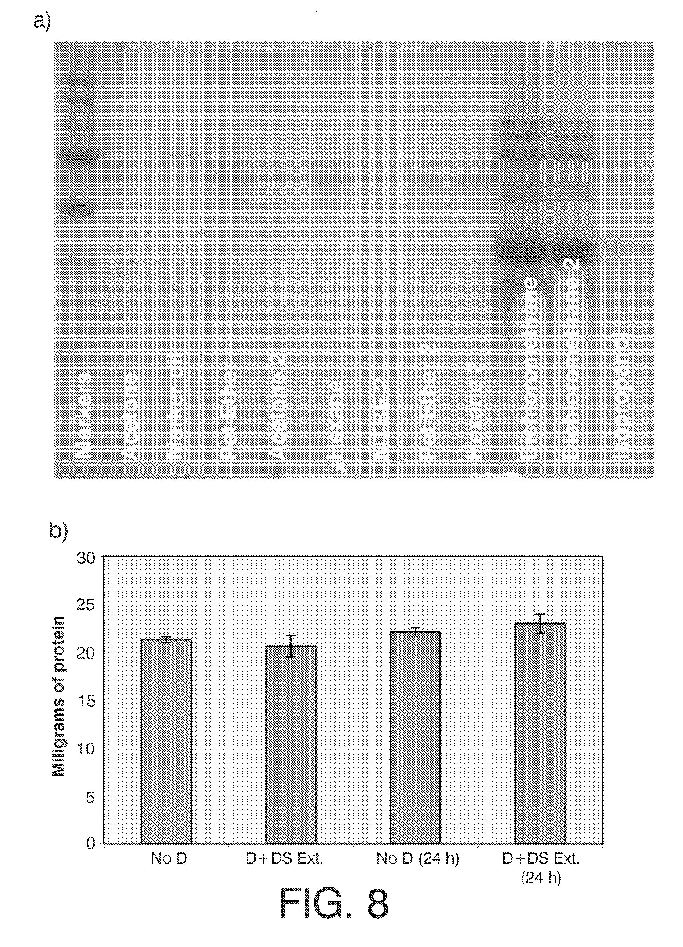

In another experiment the use of defatting agents was tested. An electrophoresis of different defatting agents used to test their effect on extraction of the gluten proteins was carried out (figure 8(a)). 125 mg of the sample was weighed and added to 1.25 ml of each defatting agent. After vortexing for 30 seconds, the solution was left to settle for 5 minutes at room temperature and centrifuged for 15 minutes and electrophoresis carried out as normal. An extraction and Bradford detection of protein in flour after the extracting defatted step without drying the sample, drying the sample for 24h was developed (figure 8 (b)). The extraction procedure was carried out, weighing the sample (75 mg), adding 0.75 ml of extraction solution, vortexing for 30 seconds, mixing in a thermomixer at 1400 rpm at 85ºC and centrifuging for 3 minutes at 12000 rpm at room temperature.

It was shown that acetone did not extract gliadins or glutenins, and can thus be used as a defatting agent. Results also showed that defatting can be followed directly by extraction, without a need to dry the sample.

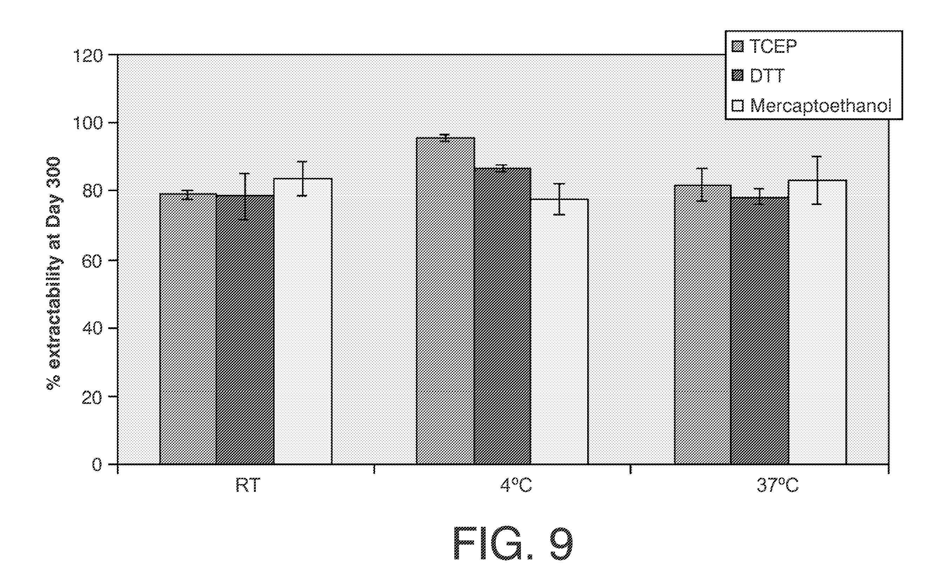

Further, stability studies of the extraction solution using DTT, mercaptoethanol or TCEP, and 60% (v/v) glycerol in buffer, using 125 mg of flour in 1.25 ml of extraction solution were carried out. The three extraction solutions were stored at RT, 4ºC and 37ºC (figure 9). These studies outline the real time stability at RT and 4°C and accelerated stability (at 37°C) of the extraction cocktail with either DTT/2-ME or 60% glycerol v/v, in 50mM HEPES buffer, pH 7.4, and the TCEP is markedly more stable when stored at 4 °C.

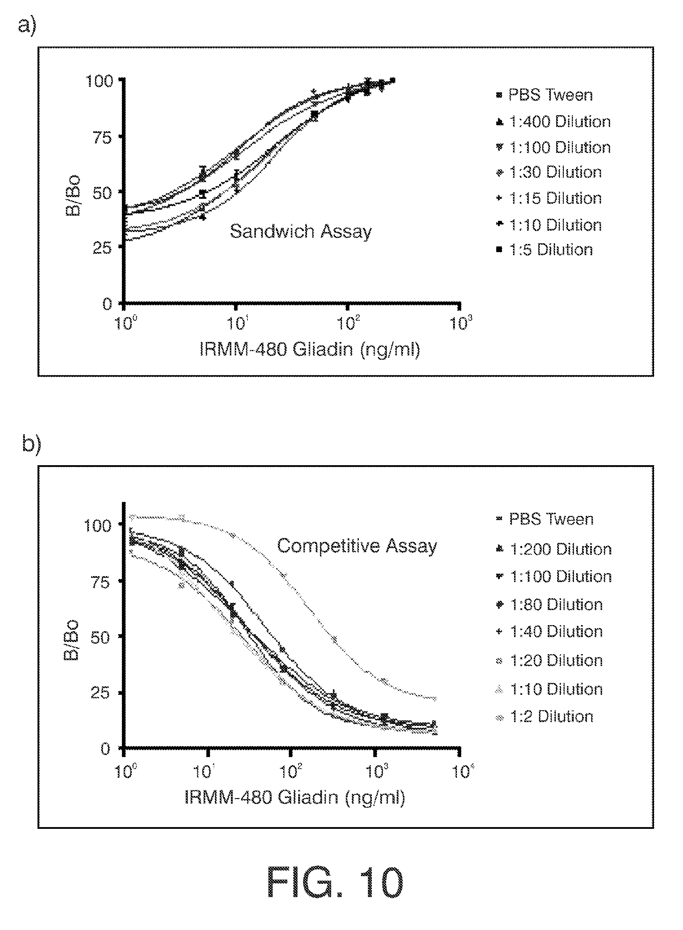

Finally, an ELISA assay, developed by the authors, based on using antibodies against the putative immunodominant epitope (Figure 10) demonstrated that the extraction solution of the invention is extremely compatible with both sandwich and competitive ELISAs. The results shown in Figure 10 are normalized by relating the absorbance at a given concentration of gliadin, with the absorbance obtained at zero concentration in the case of the competitive assay, or with the absorbance obtained at maximum concentration (e.g. 1 g/ml) in the case of the sandwich assay. In both assays the extraction buffer is completely compatible at dilutions of 1 in 5 and greater.

### Example 1. Recovery of gluten from non-heat treated foods

This experiment was carried out to show the efficiency of the process of the invention in the extraction of gluten from non-heat treated foods.

### Preparation of spiked samples

Studies were carried out with raw samples to check the efficiency of the developed extraction protocols. Corn flour was spiked with IRMM-480 prolamin working group gliadin in a range of concentrations from 20 ppm to 800 ppm. ELISA was then used to quantify the extracted gliadin using IRMM-480 gliadin as the standard, as this was the gliadin that had been used to spike the samples. The standards used varied between 25 ppm and 1.56 ppm and the spiked samples thus needed to be diluted to fall into this range. The sample dilutions employed are shown in Table 2:

**Table 2. Sample dilutions**

| | | **1^{st} extraction** | **2^{nd} extraction** |
|---|---|---|---|
| Spiked | 0ppm: | 1/150 | 1/50 |
| Spiked | 20ppm: | 1/300 | 1/100 |
| Spiked | 50ppm: | 1/800 | 1/200 |
| Spiked | 100ppm: | 1/1600 | 1/400 |
| Spiked | 200ppm: | 1/3000 | 1/800 |
| Spiked | 400ppm: | 1/6000 | 1/1600 |
| Spiked | 800ppm: | 1/12000 | 1/3000 |

### Process for simultaneous extraction of gluten from non-heat treated foods

To carry out the process, 125 mg of homogenised sample was introduced into an eppendorf tube. Subsequently, 1.25 ml of the composition of the process, comprising of 0.1% w/v TCEP in 60% v/v glycerol with 50mM HEPES buffer, pH 7.4 followed by a rapid 30 second vortex at 2500 rpm (VELP Scientifica, Madrid, Spain). The tubes were then closed and sealed with Parafilm to prevent any loss due to evaporation and mixed in a thermomixer at 95°C for 5 minutes (Thermomixer Compact model), before finally being centrifuged (Eppendorf 5417R model) at 12000 rpm at room temperature for 3 minutes. Thus, extraction could be completed in less than 10 minutes.

The supernatant was then transferred to clean polysulphone eppendorf tubes for analysis using ELISA.

HEPES buffer pH 7.4 was prepared by adding 4 g of NaOH from Panreac (VidraFoc, Tarragona, Spain) to 1.19 g of HEPES (Sigma, Barcelona, Spain), in 100 ml Millipore water.

The composition comprised of 0.1% w/v TCEP and 50 mM HEPES buffer, is prepared, for example by weighing 286.65 mg of TCEP.HCl in 10 mL of 50 mM HEPES buffer, and then adding this to a previously prepared solution of 60mL glycerol with 30 ml of 50 mM HEPES buffer, to a final volume of 100 ml, with 0.01% w/v TCEP in 60% v/v glycerol in 50 mM HEPES, pH 7.4.

### Results

Two extraction steps were required for complete extraction of gluten from raw foodstuffs, and a study was carried out to see if there was a correlation factor between the amount of protein extracted in the first extraction and the total extractable protein. In a study of 100 samples, it was observed that the protein extracted in the first extraction was 86.5% of the total extractable protein, with an RSD% of 1.28 for n=100, suggesting that one extraction could be employed and a correlation factor used to extrapolate to the total protein present in the sample. It is thus recommendable to carry out two extractions, or, to use the correlation factor of 1.16 (i.e. multiply the value of the quantity of protein extracted in the first extract by 1.16) to extrapolate the protein extracted in the first extraction to the total amount of extractable protein.

Table 3 shows the percentage recoveries achieved with the corn flour samples spiked with the PWG, and acceptable ranging between 99 and 108% (particularly if one allows for the ±15% error commonly associated with ELISA detection).

**Table 3 Percentage recoveries of raw spiked corn-flour samples**

| **Concentration of Gliadin Spiked (ppm)** | **Concentration of protein extracted and detected (ppm)** | **Percentage Recovery (%)** |
|---|---|---|
| 20 | 21.50 | 107.50 |
| 50 | 53.11 | 106.22 |
| 100 | 99.99 | 99.99 |
| 200 | 195.96 | 97.98 |
| 400 | 430.96 | 10.74 |
| 800 | 796.14 | 99.52 |

### Example 2. Recovery of gluten from heat-treated foods

This experiment was carried out to demonstrate that in the absence of a dissociating agent, an increase in temperature above 80°C is required, thus necessitating the use of a solvent with high boiling temperatures. The demonstration was carried out using heat processed samples prepared (a) from gluten containing flour and (b) cornflour.

### Materials

### - Heat processed samples from gluten containing flour

Heat processed samples from gluten containing flour were prepared by adding 5 ml of distilled water to 10 mg of raw sample i.e. wheat flour. The solution was hand mixed until a flexible dough was obtained. The dough was rolled using a hand roller to a thickness of 2 mm and cooked in a pre-heated oven for 10 minutes at 180 ºC. After cooling, the cooked samples were milled with a whisk for 3 minutes until a homogenous powder of heat processed sample was obtained.

### - Heat processed samples from gluten free corn flour spiked with IRMM-480 gliadin (IRMM = Institute for Reference Materials and Measurements)

Pre-cooked white corn flour (P.A.N. Venezuela), expected to be gluten free, and was spiked with different amounts of IRMM-480 gliadin. For 0, 20, 50, 100, 200, 400, 800 ppm of gliadin in flour, respectively, 0, 1, 2.5, 5, 10, 20 and 40 mg of IRMM-480 PWG gliadin was added to corn flour until a final quantity of 50 g was obtained. Briefly, the flour was added, very gradually, to the weighed gliadin and mixed thoroughly for 10 minutes with a pestle and mortar in order to obtain the most homogenous mixture possible. Once good representative spiked samples had been obtained, 10 mg of sample was added to 5 mL of distilled water, and was mixed by hand until a flexible dough was obtained. The dough was then rolled using a hand roller to a thickness of about 2 mm, and was then cooked at 180 ºC for 10 minutes. When the heat processed samples were cool, they were milled for 3 minutes using a Moulinex commercial blender, until a homogenous powder of heat processed sample was obtained.

### Process of the invention

To carry out the process, 125 mg of homogenised sample was introduced into an eppendorf tube. Subsequently, 1.25 ml of the composition of the process, comprising of 0,1% w/v TCEP in 60% v/v glycerol with 50 mM HEPES buffer, pH 7.4 followed by a rapid 30 second vortex at 2500 rpm (VELP Scientifica, Madrid, Spain). The tubes were then closed and sealed with Parafilm to prevent any loss due to evaporation and mixed in a thermomixer at 95 ºC for 5 minutes (Thermomixer Compact model), before finally being centrifuged (Eppendorf 5417R model) at 12000 rpm at room temperature for 3 minutes. Thus, extraction could be completed in less than 10 minutes.

The supernatant was then transferred to clean polysulphone eppendorf tubes for analysis using ELISA.

HEPES buffer pH 7.4 was prepared by adding 4 g of NaOH from Panreac (VidraFoc, Tarragona, Spain) to 1.19 g of HEPES (Sigma, Barcelona, Spain), in 100 mL Millipore water.

The composition comprised of 0.1% w/v TCEP and 50 mM HEPES buffer, is prepared, for example by weighing 286.65 mg of TCEP. HCl in 10 mL of 50 mM HEPES buffer, and then adding this to a previously prepared solution of 60 mL glycerol with 30 mL of 50 mM HEPES buffer, to a final volume of 100 mL, with 0.01 % w/v TCEP in 60% v/v glycerol in 50 mM HEPES, pH 7.4.

### Results

### - Effect of temperature on extraction from non-heat and heat processed samples

A Gel electrophoresis analysis of gluten extracted at room temperature using 0.1 % w/v TCEP in 60% v/v glycerol with 50mM HEPES buffer, pH 7 for non-heat processed samples (raw gluten-containing wheat flour) and heat-processed samples (the same gluten-containing wheat flour prepared as pizza base and cooked at 180 ºC for 10 minutes) was carried out.

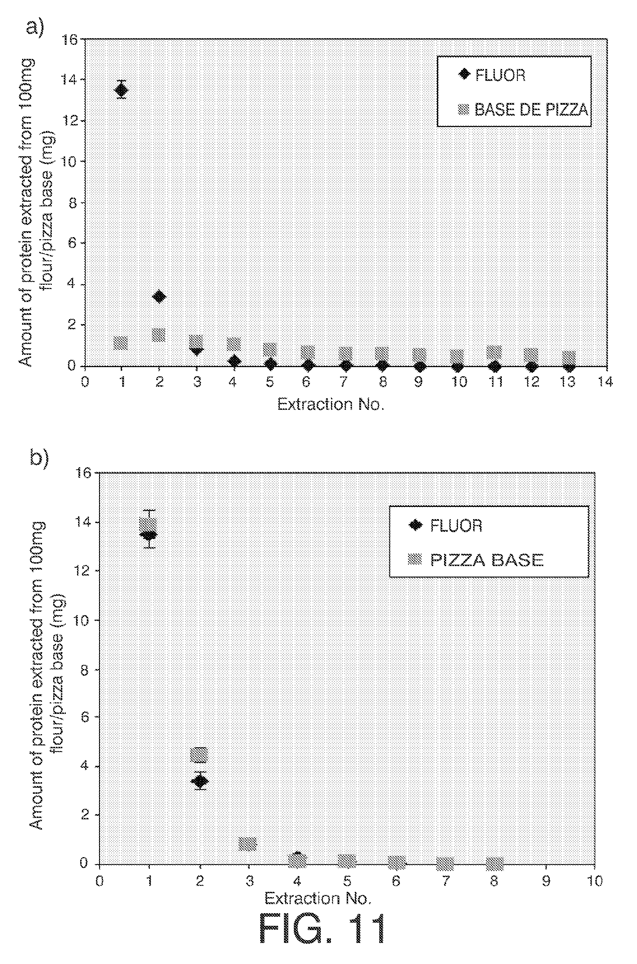

Figure 11 shows the requirement for increasing the extraction temperature to efficiently extract from heat processed samples. At room temperature, the gluten was extracted in two extractions from non-heat processed gluten containing flour. However, for the heat processed samples ("pizza base"), after 13 extractions the gluten had not been completely extracted, with just a small fraction of the gluten being released for each extraction.

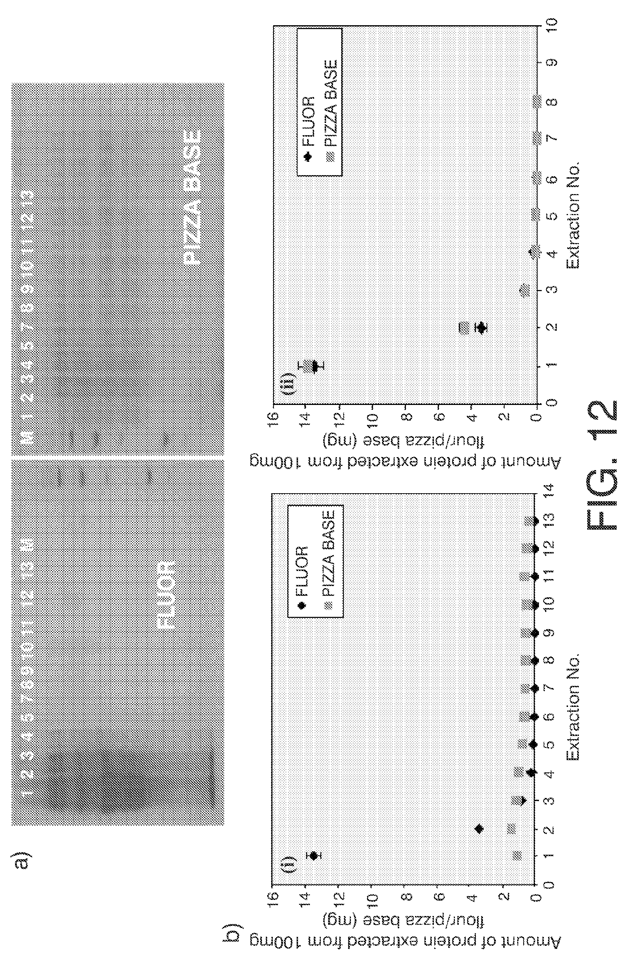

In figure 12, Gluten extracted at each extraction from non-heat processed ("flour") and heat-processed samples ("pizza base"), with extraction being carried out at room temperature (a) or at 85 ºC (b). Complete extraction was achieved at 85 ºC with just two extractions.

It was shown that by increasing the extraction temperature from room temperature to 85 ºC, the total gluten can be extracted from both the raw and heat-processed food samples in the first two extractions.

### - Number of extractions and correlation factor

Two extractions are required for complete extraction of gluten from cooked foodstuffs, as in the case of the raw foodstuffs, with more HMW glutenins being extracted in the second extraction. Again, as in the case of the raw foodstuffs, there is a correlation factor between the amount of protein extracted in the first extraction with respect to the total extractable protein, which is 58.1, n=400, RSD%= 7.87. Thus, for cooked samples, the correlation factor requires to multiply the value of the quantity extracted in the first extract by 1.72.

### - Recovery of gluten from heat processed corn flour spiked with gluten

Table 4 shows the percentage recoveries achieved with the corn flour samples spiked with the PWG, and acceptable ranging between 96 and 111% (particularly if one allows for the ±15% error commonly associated with ELISA detection).

**Table 4 Percentage recoveries of heat processed spiked corn-flour samples**

| **Concentration of Gliadin Spiked (ppm)** | **Concentration of protein extracted and detected (ppm)** | **Percentage Recovery (%)** |
|---|---|---|
| 20 | 22.27 | 111.35 |
| 50 | 48.26 | 96.52 |
| 100 | 97.24 | 97.24 |
| 200 | 201.34 | 100.67 |
| 400 | 423.86 | 105.97 |
| 800 | 797.22 | 99.65 |

### Example 3 Comparison of the process of the invention with the extraction protocol of patent ES2182698

The objective of this Example is to compare the extraction efficiency of the invented protocol and composition (Extraction Protocol A) with the method and protocol described in Patent Application ES2182698 (Extraction Protocol B).

### Materials

Preparation of spiked samples (gluten free): Spiked samples were prepared mixing different concentrations of PWG gliadin diluted in 60% v/v ethanol with Maizena corn flour. For 0, 20, 25, 50, 100, 150 ppm of gliadin in flour, 0, 1, 1.25, 2.5, 5, 7.5 mg of PWG gliadin was diluted in 25 ml of 60%v/v ethanol in 50mM HEPES pH 7.4 and mixed with the corn flour reaching a total of 50 g solid in the 25ml. Following thorough homogenization, the samples were dried at room temperature. The spiked samples were extracted using Extraction Protocol A and B, and analysed using the Ingezim Gluten ELISA Kit.

Preparation of heat processed samples from gluten containing wheat flour: Heat processed samples from gluten containing flour were prepared by adding 5 ml of distilled water to 10 mg of raw sample i.e. wheat flour. The solution was hand mixed until a flexible dough was obtained. The dough was rolled using a hand roller to a thickness of 2 mm and cooked in a pre-heated oven for 10 minutes at 180ºC. After cooling, the cooked samples were milled with a whisk for 3 minutes until a homogenous powder of heat processed sample was obtained.

### Method

Extraction Protocol A: 125 mg of homogenised sample was introduced into an eppendorf tube. Subsequently, 1.25 ml of the composition of the process, comprising of 0.1% w/v TCEP in 60% v/v glycerol with 50mM HEPES buffer, pH 7.4 followed by a rapid 30 second vortex at 2500 rpm (VELP Scientifica, Madrid, Spain). The tubes were then closed and sealed with Parafilm to prevent any loss due to evaporation and mixed in a thermomixer at 95 ºC for 5 minutes (Thermomixer Compact model), before finally being centrifuged (Eppendorf 5417R model) at 12000 rpm at room temperature for 3 minutes. Thus, extraction could be completed in less than 10 minutes.

Extraction Protocol B consisted of weighting 250 mg sample and adding 2.5 ml of 250mM 2-mercaptoethanol and 2M guanidine hydrochloride in 80% v/v ethanol and vortexing for 5 seconds. After mixing for 1 hour at room temperature, 7.5 ml of ethanol 80% v/v in deionised H₂O was added, mixed for 1 hour more and centrifuged for 10 minutes at 12.000 rpm.

For both Extraction Protocols, the supernatant was then transferred to clean polysulphone eppendorf tubes for analysis using ELISA.

HEPES buffer pH 7.4 was prepared by adding 4 g of NaOH from Panreac (VidraFoc, Tarragona, Spain) to 1.19g of HEPES (Sigma, Barcelona, Spain), in 100 mL Millipore water

The composition of Extraction Protocol A comprised of 0.1% w/v TCEP and 50 mM HEPES buffer, is prepared, for example by weighing 286.65 mg of TCEP. HCl in 10 ml of 50 mM HEPES buffer, and then adding this to a previously prepared solution of 60 ml glycerol with 30 ml of 50 mM HEPES buffer, to a final volume of 100 ml, with 0.01% w/v TCEP in 60% v/v Glycerol in 50 mM HEPES, pH 7.4.

It was also attempted to increase the extraction temperature of the first step of Extraction Protocol B, to the same temperature as that used in Extraction Protocol A (e.g. 85 ºC), but due to the low boiling point of ethanol the temperature could only be increased to 60 ºC.

### Results

Comparing the percentage recoveries from heat processed samples, achieved using the Extraction protocols, and analysing using a commercial R5 Sandwich ELISA kit, which detects a repeating pentamer sequence found on gliadin, the recoveries are comparable, with the process of this invention demonstrating better recovery at lower concentrations.

**Table 5 Comparison of extraction recoveries using the process of the invention and the invention according to US2004/0137137A1**

| | **EXTRACTION PROTOCOL A** | |
|---|---|---|
| **Concentration of Gliadin Spiked (ppm)** | **Concentration of protein extracted and detected (ppm)** | **Percentaje recovery (%)** |
| 0.00 | 0.48 | - |
| 20.00 | 18.58 | 92.90 |
| 25.00 | 27.37 | 109.48 |
| 50.00 | 55.22 | 110.44 |
| 100.00 | 96.97 | 96.97 |
| 150.00 | 165.10 | 110.07 |

| **EXTRACTION PROTOCOL B** | | |
|---|---|---|
| **Concentration of Gliadin Spiked (ppm)** | **Concentration of protein extracted and detected (ppm)** | **Percentaje recovery (%)** |
| 0.00 | 0.59 | - |
| 20.00 | 13.46 | 67.30 |
| 25.00 | 25.24 | 100.96 |
| 50.00 | 44.48 | 88.96 |
| 100.00 | 95.34 | 95.34 |
| 150.00 | 142.62 | 95.08 |

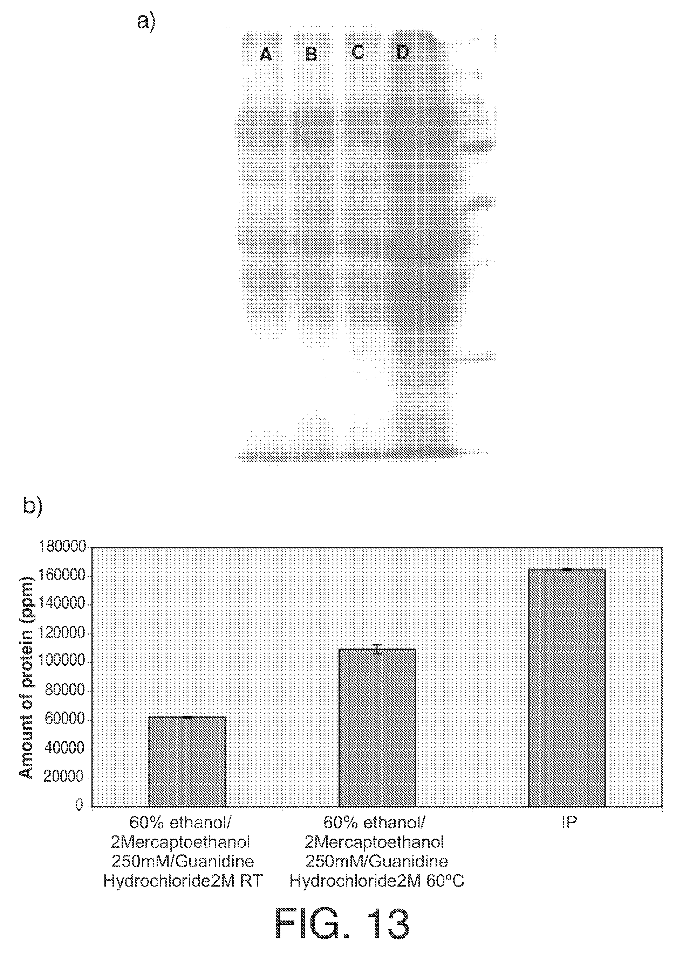

Additionally, the extraction from heat-processed samples was compared using Bradford analysis and electrophoresis. For this demonstration gluten containing wheat flour was used to prepare heat processed samples. As shown in Figure 13.a), almost three times more protein (in terms of total protein) is extracted using Extraction Protocol A, which extracts 4.21 mg from a 25 mg samples of pizza base as compared to 1.47 mg extracted using Extraction Protocol B. This is most probably not measured using the Ingezim R5 Sandwich ELISA, as the main difference in what is being extracted is principally LMW and HMW glutenins. The effect of increasing the temperature of extraction of Protocol B was also compared, and as shown in Figure 13.a), enhanced extraction of the HMW glutenins was achieved, with 2.66 mg total protein being extracted as compared to the 1.47 mg extracted at room temperature.

The electrophoresis of extraction of gluten proteins (figure 13.a) was carried out using (A) 60% ethanol, 250 mM mercaptoethanol, 2M Guanidine hydrochloride, with extraction carried out at room temperature (B) 60% ethanol, 250 mM mercaptoethanol, 2M Guanidine hydrochloride, with extraction carried out at 60°C and (C) Invented Protocol, Extraction 1 (D) Invented Protocol, Extraction 2.

### Example 4. Gluten extraction from a range of commercially available gluten-free samples, and hydrolyseds

### Materials

The commercial samples studied can be divided into four groups:
- Gluten containing samples
- Gluten free food samples
- Gluten free food samples requiring defatting
- Gluten hydrolyseds

Sampling for each sample, five samples from different parts of the food sample were taken and combined to a 1 g quantity, which was taken and then thoroughly homogenized using a Moulinex blender. Representative sampling was then used to provide a homogenous sample, for analysis.

Defatting of samples: In the case of the samples that required defatting, 1.25ml of acetone was added to the homogenised sample (125 mg) and vortexed for 30 seconds at room temperature, allowed to settle for 5 minutes and centrifuged for 15 minutes at 14000 rpm at room temperature. Immediately after defatting the pellet obtained following centrifugation was applied to the process of the invention.

125 mg of homogenised sample was introduced into an eppendorf tube. Subsequently, 1.25 ml of the composition of the process, comprising of 0.1%w/v TCEP in 60% v/v glycerol with 50 mM HEPES buffer, pH 7.4 followed by a rapid 30 second vortex at 2500 rpm (VELP Scientifica, Madrid, Spain). The tubes were then closed and sealed with Parafilm to prevent any loss due to evaporation and mixed in a thermomixer at 95°C for 5 minutes (Thermomixer Compact model), before finally being centrifuged (Eppendorf 5417R model) at 12000rpm at room temperature for 3 minutes. Thus, extraction could be completed in less than 10 minutes.

The supernatant was then transferred to clean polysulphone eppendorf tubes for analysis using ELISA. Buffer pH 7.4 was prepared by adding 4 g of NaOH from Panreac (VidraFoc, Tarragona, Spain) to 1.19 g of HEPES (Sigma, Barcelona, Spain), in 100 mL Millipore water. The composition of Extraction Protocol A comprised of 0,1% w/v TCEP and 50 mM HEPES buffer, is prepared, for example by weighing 286.65 mg of TCEP.HCl in 10 mL of 50 mM HEPES buffer, and then adding this to a previously prepared solution of 60 mL glycerol with 30 mL of 50 mM HEPES buffer, to a final volume of 100 mL, with 0.01% w/v TCEP in 60% v/v glycerol in 50 mM HEPES, pH 7.4.

**Table 6 Gliadin extracted and detected using in-house ELISA**

| ***Gluten Containing Samples (defatted)*** | **Concentration of Gliadin (ppm)** |
|---|---|
| Sponge Cake | >10000 |
| BBQ Sauce | 363.74 |
| Cookie flavoured yoghurt | 95.64 |
| Strawberry flavoured yoghurt | 46.13 |
| Light Mayonnaise | 115.78 |
| | |

| ***Gluten-free samples (non-defatted)*** | **Concentration of Gliadin (ppm)** |
|---|---|
| Peach Jam | 1.01 |
| Cream of balsamic vinegar | 0.88 |
| | |

| ***Gluten-free samples (defatted)*** | **Concentration of Gliadin (ppm)** |
|---|---|
| Vegetable margarine | 1.16 |
| Whipped Cream | 7.13 |
| Grated Cheese | 1.59 |
| Potato snacks | 0.89 |
| Chorizo sausage | 1.60 |
| Frankfurt sausage | 1.58 |
| Black Pudding Sausage | 4.26 |
| Panceta | 1.87 |
| Tomato puree | 3.76 |
| | |

| ***Gluten hydrolysates (non-defatted)*** | **Concentration of Gliadin (ppm)** |
|---|---|
| Mustard | 3.89 |
| Chocolate Syrup | 2.55 |
| Aspartame | 1.70 |
| Instant Cocoa | 1.63 |
| Maria Rose Sauce | 3.54 |
| Wheat Starch # 1 | 29.40 |
| Wheat Starch # 2 | 23.45 |
| Wheat Starch # 3 | 80.12 |
| Wheat Starch # 4 | 114.27 |
| Wheat Starch # 5 | 80.70 |
| Wheat maltodextrin | 0.14 |
| Wheat dextroxe | 0.00 |
| Flakes of corn (breakfast cereal) | 5701.75 |
| Malt flavouring 20606 | 280.66 |
| Malt flavouring 20606 | 363.41 |
| Beer # 1 | 317.11 |
| Beer # 2 | 133.66 |
| Beer # 3 | 429.99 |

### BIBLIOGRAPHY

1. Sollid L M & Thorsby E. Gastroenterology (1993),105, 910-22
2. Catassi C, Rossini M, Ratsch I-M, Bearzi I, Santinelli A, Castagnani R, Pisani E, Coppa GV & Giorgi PL. 1993, Gut, 34, 1515- 9
3. Faulkner-Hogg KB, Selby WS, Loblay RH. (1999), Scand J Gastroenterol, 34 784-9
4. Holmes GKT, Prior P, and Lane MR et al. (1989), Gut, 30, 333 - 8
5. Ellis HJ, Rosen-Bronson S, O'Reilly N and Ciclitira PJ. (1998), Gut, 43, 190 - 5
6. Skerrit, JH & Hill AS. (1991), Journal of the Association of Official Analytical Chemistry, 74 257 - 264
7. Sorell L, Lopez J A, Valdes I, Alfonso P, Camafeita E, Acevedo B, Chirdo F, Galvilondo J, Mendez E. (1998), FEBS Letters, 439, 46 - 50
8. Aubrecht E & Toth A. (1995), Acta Alimentaria, 24, 23 - 29
9. Troncone R, Vitale M, Donatiello A, Farris E, Roos G & Auricchio S. (1986), Journal of Immunological Methods, 92, 21-23
10. Chirdo FG, Anon MC & Fossati CA. (1998), Food & Agricultural Immunology, 10, 143-155
11. Skerritt J H and Hill A S. Journal of Agricultural and Food Chemistry, (1990), 38, 1771 -1778
12. Ellis H J, Doyle A P, Wieser H, Sturgess R P, Day P and Ciclitira P J. (1994), Journal of Biochemical and Biophysical methods, 28, 77 - 82
13. Chirdo F G, Anon M C and Fossati C A. (1995), Food and Agricultural Immunology, 7, 333 - 343
14. Nicolas Y, Denery-Papini S, Martinant J P and Popineau Y. (2000), Food and Agricultural Immunology, 12, 53-65
15. Valdes I, Garcia E, Llorente M, Mendez E. (2003), Eur J Gastroenterol Hepatol, 155, 465 -474
16. Spaenij-Dekking EHA, Kooy-Winkelaar EMC, Nieuwenhuizen WF, Drijifhout JW, Koning F. (2004), Gut, 53, 1267 - 1273
17. Dewar D, Pereira SP, Ciclitira PJ. (2004), Int J Biochem Cell Biol. 36,17-24.
18. Shan L, Molberg O, Parrot I, Hausch F, Filiz F, Gray GM, Sollid LM, Khosla C. (2002) Science, 29727, 2275 - 2279
19. Bietz, J.A. (1984), Bakers Dig. 58(1), 15-17, 20-21, 32
20. Bietz, J.A. (1983), Journal of Chromatography, 255, 239-245
21. Huebner, F.R. and Bietz, J.A., (1993), Cereal Chemistry, 70, 506-511.
22. Wieser, H., Seilmeier, W. and Belitz, H.D., (1994), Journal of Cereal Science, 19, 149-155
23. Lookhart, G.L., Jones, B.L., Hall, S.B., and Finney, K.F., (1982), Cereal Chemistry, 59, 178-181
24. Marchylo, B.A. and Kruger, J.E., (1988), Cereal Chemistry, 65, 192-198
25. Lookhart, G. and Bean, S., (1995), Cereal Chemistry, 72, 42-47
26. Aubrecht, E., and Tóth, A., (1995), Acta Alimentaria, 24, 23-29
27. Burnouf, T. And Bietz, J.A., (1989), Cereal Chemistry, 66, 121-127
28. Melas, V., Morel, M.H., Autran, J.C., and Feillet, P., (1994), Cereal Chemistry, 71, 234-237
29. Fu, B.X. and Sapirstein, H.D., (1996), Cereal Chemistry, 73, 143-152
30. Marchylo, B.A., Hatcher, D.W., Kruger, J.E., and Kirkland, J.J., (1992), Cereal Chemistry, 69, 371-378
31. Byers, M., Miflin, B.J. and Smith, S.J., (1983), J.Sci.Food.Agric, 34, 447-462
32. Wieser, H., Antes, S., Seilmeier, W., (1998), Cereal Chemistry, 75, 644-650
33. Marchylo, B.A:, Kruger, H.E., and Hatcher, D.W., (1989), Journal of Cereal Science, 9, 113-130
34. Kazemie, M. and Bushuk, W., (1992), Cereal Chemistry, 69, 105-107
35. Burnouf, T. and Bietz, J.A., (1989), Cereal Chemistry, 66, 121-127
36. Gupta, R.B. and MacRitchie, F., (1991), Journal of Cereal Science, 14, 105-109
37. Bietz, J.A., Burnouf, T., Conn, L.A. and Wall, J.S., (1984), Cereal Chemistry, 61, 124-129
38. Nicolas, Y., Larré, C., and Popineau, Y., (1997), Journal of Cereal Science, 25, 151-154
39. Sutton, K.H., (1991), Journal of Cereal Science, 14, 25-34
40. Hou, G. and Ng, P.K.W, (1995), Cereal Chemistry, 72, 545-551
41. Morel, M.H., (1994), Cereal Chemistry, 71, 238-242
42. Singh, N.K., Shepherd, K.W., and Cornish, G.B., (1991), Journal of Cereal Science, 14, 203-208
43. Marhcylo, B.A., Hatcher, D.W., and Kruger, J.E., (1988), Cereal Chemistry, 65, 28-40
44. Keck, B., Köhler, P., and Wieser, H., (1995), Z Lebensm Unters Forsch, 200, 432-439
45. Burnouf, T. and Bietz, J.A., (1984), Journal of Chromatography, 299, 185-199
46. Mimouni, B., Robin, J.M., Azanza, J.L., and Raymond, J., (1998), J Sci Food Agric, 78, 423-428
47. Tkachuk, R., and Mellish, V.J., Paper No. 600 of the Grain Research Laboratory, Canadian Grain Commission
48. Garcia, E., Llorente, M., Hernando, A., Kieffer, R., Wieser, H., and Mendez, E., (2005), Eur. J. Gastroenterol & Hepatol, 17, 529-539
49. Consejo Superior de Investigaciones Cientificas. "Procedimiento para la extacción de gluten en alimentos, procesados y no procesados por calor, compatible con ELISA, composición y kits que comprenden dicha composición". WO02092633, November 21 th, 2002.
50. Consejo Superior de Investigaciones Científicas. "Procedimiento para la extacción cuantitativa de gluten en alimentos procesados o no procesados por calor, y composición solubilizante de proteínas de gluten o un kit comercial que la contenga necesario para su puesta en practica". ES2182698, May, 13 the 2004. Appl. 200101098, May 14 th, 2001.
51. Wieser, H. Food Research and Technology (1998), 207(2), 128-132.
52. Wieser H., Antes,S., and Seilmeier W., (1998) Cereal Chem, 75(5), 644-650.
53. Nicolas Y. Martinant, J.-P., Denery-Papini, S., Popineau, Y. (1998) J. Sci. Food Agric, 77, 96-102
54. Huebner F.R. and Bietz, J.A., (1999) Cereal Chem. 76(2), 299-302.
55. Mamone G., Ferranti, P., Chianese, L., Scafuri, L., Addeo, F. (2000), Rapid Commun. Mass Spectrom 14, 897-904
56. Consejo Superior de Investigaciones Científicas. "Method for extracting gluten from processed and unprocessed foods by means of heat based on the use of ionic and non-ionic detergents". WO2007/104825, September 20th, 2007. Priority number ES200600675, March, 16 th, 2006.
57. Hurkman, W.J. and Tanaka, C.K., (2007), Journal of Cromatography B, 849, 344-350
58. DuPont, F.M., Chan, R., Lopez, R., Vensel, W.H., (2005), Journal of Agricultural and Food Chemistry, 53, 1575-1584.
59. Burns, J.A., Butler, J.C., Moran, J., Whitesides, G.M., (1991), J.Org.Chem., 56, 2648-2650
60. Getz, E.B., Xiao, M., Chakrabarty, T., Cooke, R., and Selvin, P.R., (1999), Anal. Biochem., 273, 73-80
61. Shafer, D.E., Inman, J.K., and Lees, A., (2000), Anal. Biochem., 282, 161-164
62. Lykkesfeldt, J., (2000), Anal. Biochem., 282, 89-93
63. Krezel, A., Latajka, R., Bujacz, G.D., and Bal., W., (2003), Inorganic Chemistry, 42, 1994-2003
64. Carmen Bermudo et al (2005) (65) Monoclonal antibody based competitive assay for the sensitive detection of coeliac disease toxic prolamins, Anal-Chim.Acta, 551, 105-114.

## Claims

1. A composition that consists of a reducing agent for the irreversible reduction of disulphide agents and a solvent with high boiling point and/or high dielectric constant in a buffer with a pH between 4 and 9.

2. A composition according to claim 1, wherein the reducing agent is a non-toxic reducing agent with a concentration greater than 0.1 mM.

3. A composition according to claim 2, wherein the reducing agent is Tris (2-carboxyethyl) phosphine (TCEP).

4. A composition according to claim 1, wherein the solvent is selected from DMF, DMSO or glycerol, with a concentration between 30-70% v/v.

5. A composition according to any of claims 1-4, that consists in TCEP, as the reduction agent, a solvent selected from glycerol, DMSO or DMF in HEPES or PBS buffer with a pH between 4 and 9.

6. A process for simultaneous extraction of gliadins and glutenins, from both heat processed and non-heat processed food samples that comprises extracting the gliadins and glutenins content in the presence of a composition according to any of claims 1-5.

7. A process according to claim 6 that comprises: a) mixing the food sample with a composition according to any of claims 1-5, and b) incubating the resulting mixture at a temperature greater than 50 ºC, for a period of time of greater than 2 minutes.

8. A process according to any of claims 6 and 7 that comprises repeating this process in a range of 2 to 10 times.

9. A process according to any of claims 6-8 wherein, previous to the extraction, a defatting step is carried out.

10. A process according to claim 9, wherein the defatting step is carried out using acetone.

11. A process according to any of claims 6-10 wherein, after the extraction, a quantification step of the extracted gliadins and glutenins using ELISA is carried out.

12. A kit that comprises a composition according to any of claims 1 to 5.

13. A kit according to claim 12 that further comprises the necessary reagents to perform an ELISA to quantify the gliadins and glutenins contents of both heat processed and non-heat processed food samples.
